# EUROPEAN PATENT APPLICATION

(11) **EP 4 541 376 A1**
(43) Date of publication of application: **23.04.2025**
(21) Application number: 23204220.0
(22) Date of filing: 17.10.2023
(51) Int. Cl.: A61K 47/68

(54) **ANTIBODY-DRUG CONJUGATES**

(71) Applicant: ADC Therapeutics SA, 1066 Epalinges (CH)
(72) Inventor: The designation of the inventor has not yet been filed
(74) Representative: Bridle, Andrew Barry

(57) **Abstract**

The present disclosure relates to drug-linker systems for antibody-drug conjugates, and antibody-drug-conjugate comprising the same.

## Description

### Field of the invention

The present invention relates to the field of antibody-drug conjugates, in particular to antibody-drug conjugates with a polyglycine linker.

### Background of the invention

Antibody-drug conjugates (ADC), are essentially targeted delivery of an active pharmaceutical drug to a specific cellular location of choice is a powerful approach for the treatment of a wide range of diseases, with many beneficial aspects versus systemic delivery of the same drug.

In the field of ADCs, a chemical linker is typically employed to attach a pharmaceutical drug to an antibody. This linker needs to possess a number of key attributes, including the requirement to be stable in plasma after drug administration for an extended period of time. A stable linker enables localization of the ADC to the projected site or cells in the body and prevents premature release of the payload in circulation, which would indiscriminately induce undesired biological response of all kinds, thereby lowering the therapeutic index of the ADC. Upon internalization, the ADC should be processed such that the payload is effectively released so it can bind to its target.

### Summary

The present inventors have surprisingly found that linkers with a polyglycine system are highly suitable for metal-free click or thiol conjugation of drug payloads to antibodies, resulting in antibody-drug conjugates that show no or negligible aggregation propensity. The resulting ADCs were found to display significant *in vivo* efficacy. As such, the inventors have for the first time been able to prepare an ADC with payloads that exhibits efficacy in the same order of magnitude as the most efficacious antibody-drug conjugates, in particular antibody-exatecan conjugates, known to date, while showing no aggregation at all. It was also found that the resulting ADCs displayed improved conjugation efficiency.

The invention first and foremost concerns an antibody-drug conjugate, having structure (1) 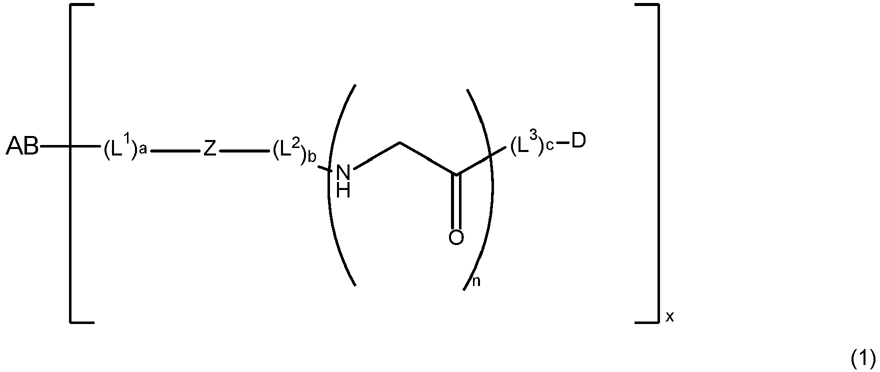 wherein:
AB is a cell binding agent, such as an antibody;
n is an integer in the range of 5 - 8;
L¹ is a linker;
L² is a linker;
L³ is a linker;
a, b and c are independently 0 or 1;
Z is a connecting group obtained by a metal-free click reaction or by thiol ligation;
or Z is absent wherein a and b are 0 and the N terminus of the polyglycine is bonded directly to AB;
D is a drug payload;
x is an integer in the range of 1 - 8.

The invention further concerns a process for the synthesis of the antibody-drug conjugate according to the invention, a linker-drug construct which is suitable to be used in the process according to the invention, the medical use of the antibody-drug conjugate according to the invention and a pharmaceutical composition comprising the antibody-drug conjugate according to the invention.

### Detailed description

### Definitions

The verb "to comprise", and its conjugations, as used in this description and in the claims is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there is one and only one of the elements. The indefinite article "a" or "an" thus usually means "at least one".

The compounds disclosed in this description and in the claims may comprise one or more asymmetric centres, and different diastereomers and/or enantiomers may exist of the compounds. The description of any compound in this description and in the claims is meant to include all diastereomers, and mixtures thereof, unless stated otherwise. In addition, the description of any compound in this description and in the claims is meant to include both the individual enantiomers, as well as any mixture, racemic or otherwise, of the enantiomers, unless stated otherwise. When the structure of a compound is depicted as a specific enantiomer, it is to be understood that the invention of the present application is not limited to that specific enantiomer.

The compounds may occur in different tautomeric forms. The compounds according to the invention are meant to include all tautomeric forms, unless stated otherwise. When the structure of a compound is depicted as a specific tautomer, it is to be understood that the invention of the present application is not limited to that specific tautomer.

The compounds disclosed in this description and in the claims may further exist as R and S stereoisomers. Unless stated otherwise, the description of any compound in the description and in the claims is meant to include both the individual R and the individual S stereoisomers of a compound, as well as mixtures thereof. When the structure of a compound is depicted as a specific S or R stereoisomer, it is to be understood that the invention of the present application is not limited to that specific S or R stereoisomer.

The compounds disclosed in this description and in the claims may further exist as exo and endo diastereoisomers. Unless stated otherwise, the description of any compound in the description and in the claims is meant to include both the individual exo and the individual endo diastereoisomers of a compound, as well as mixtures thereof. When the structure of a compound is depicted as a specific endo or exo diastereomer, it is to be understood that the invention of the present application is not limited to that specific endo or exo diastereomer.

The compounds according to the invention may exist in salt form, which are also covered by the present invention. The salt is typically a pharmaceutically acceptable salt, containing a pharmaceutically acceptable anion. The term "salt thereof means a compound formed when an acidic proton, typically a proton of an acid, is replaced by a cation, such as a metal cation or an organic cation and the like. Where applicable, the salt is a pharmaceutically acceptable salt, although this is not required for salts that are not intended for administration to a patient. For example, in a salt of a compound the compound may be protonated by an inorganic or organic acid to form a cation, with the conjugate base of the inorganic or organic acid as the anionic component of the salt.

The term "pharmaceutically acceptable" salt means a salt that is acceptable for administration to a patient, such as a mammal (salts with counter ions having acceptable mammalian safety for a given dosage regime). Such salts may be derived from pharmaceutically acceptable inorganic or organic bases and from pharmaceutically acceptable inorganic or organic acids. "Pharmaceutically acceptable salt" refers to pharmaceutically acceptable salts of a compound, which salts are derived from a variety of organic and inorganic counter ions known in the art and include, for example, sodium, potassium, calcium, magnesium, ammonium, tetraalkylammonium, etc., and when the molecule contains a basic functionality, salts of organic or inorganic acids, such as hydrochloride, hydrobromide, formate, tartrate, besylate, mesylate, acetate, maleate, oxalate, etc.

The term "protein" is herein used in its normal scientific meaning. Herein, polypeptides comprising about 10 or more amino acids are considered proteins. A protein may comprise natural, but also unnatural amino acids.

The term "antibody" is herein used in its normal scientific meaning. An antibody is a protein generated by the immune system that is capable of recognizing and binding to a specific antigen. An antibody is an example of a glycoprotein. The term antibody herein is used in its broadest sense and specifically includes monoclonal antibodies, polyclonal antibodies, dimers, multimers, multi-specific antibodies (e.g. bispecific antibodies), antibody fragments, and double and single chain antibodies. The term "antibody" is herein also meant to include human antibodies, humanized antibodies, chimeric antibodies and antibodies specifically binding cancer antigen. The term "antibody" is meant to include whole immunoglobulins, but also antigen-binding fragments of an antibody. Furthermore, the term includes genetically engineered antibodies and derivatives of an antibody. Antibodies, fragments of antibodies and genetically engineered antibodies may be obtained by methods that are known in the art.

An "antibody fragment" is herein defined as a portion of an intact antibody, comprising the antigen-binding or variable region thereof. Examples of antibody fragments include Fab, Fab', F(ab')2, and Fv fragments, diabodies, minibodies, triabodies, tetrabodies, linear antibodies, single- chain antibody molecules, scFv, scFv-Fc, multispecific antibody fragments formed from antibody fragment(s), a fragment(s) produced by a Fab expression library, or an epitope-binding fragments of any of the above which immunospecifically bind to a target antigen (e.g., a cancer cell antigen, a viral antigen or a microbial antigen).

An "antigen" is herein defined as an entity to which an antibody specifically binds.

The terms "specific binding" and "specifically binds" is herein defined as the highly selective manner in which an antibody or antibody binds with its corresponding epitope of a target antigen and not with the multitude of other antigens. Typically, the antibody or antibody derivative binds with an affinity of at least about 1×10⁻⁷ M, and preferably 10⁻⁸ M to 10⁻⁹ M, 10⁻¹⁰ M, 10⁻¹¹ M, or 10⁻¹² M and binds to the predetermined antigen with an affinity that is at least two-fold greater than its affinity for binding to a non-specific antigen (e.g., BSA, casein) other than the predetermined antigen or a closely-related antigen.

The term "substantial" or "substantially" is herein defined as a majority, i.e. >50% of a population, of a mixture or a sample, preferably more than 50%, 55%, 60%, 65%, 70%, 75%, 80%, 85%, 90%, 91%, 92%, 93%, 94%, 95%, 96%, 97%, 98%, or 99% of a population.

A "linker" is herein defined as a moiety that connects two or more elements of a compound. For example in an antibody-conjugate, an antibody and a payload are covalently connected to each other via a linker. A linker may comprise one or more linkers and spacer-moieties that connect various moieties within the linker.

A "spacer" or spacer-moiety is herein defined as a moiety that spaces (i.e. provides distance between) and covalently links together two (or more) parts of a linker. The linker may be part of e.g. a linkerconstruct, the linker-conjugate or a bioconjugate, as defined below.

A "self-immolative group" is herein defined as a part of a linker in an antibody-drug conjugate with a function is to conditionally release free drug at the site targeted by the ligand unit.

The activatable self-immolative moiety comprises an activatable group (AG) and a self-immolative spacer unit. upon activation of the activatable group, for example by enzymatic conversion of an amide group to an amino group or by reduction of a disulfide to a free thiol group, a self-immolative reaction sequence is initiated that leads to release of free drug by one or more of various mechanisms, which may involve (temporary) 1 ,6-elimination of a p-aminobenzyl group to a p-quinone methide, optionally with release of carbon dioxide and/or followed by a second cyclization release mechanism. The self-immolative assembly unit can part of the chemical spacer connecting the antibody and the payload (via the functional group). Alternatively, the self-immolative group is not an inherent part of the chemical spacer, but branches off from the chemical spacer connecting the antibody and the payload A " cathepsin cleavable peptide" is herein defined as a peptide moiety wherein the peptide bond may be cleaved via a process of proteolysis which is mediated via one or more proteases selected from the cathepsin family of proteases, in particular cathepsin B and/or cathepsin D, more particularly cathepsin B.

A "conjugate" is herein defined as a compound wherein an antibody is covalently connected to a payload via a linker. A conjugate comprises one or more antibodies and/or one or more payloads.

The term "payload" refers to the moiety that is covalently attached to a targeting moiety such as an antibody, but also to the molecule that is released from the conjugate upon uptake of the protein conjugate and/or cleavage of the linker. Payload thus refers to the monovalent moiety having one open end which is covalently attached to the targeting moiety via a linker and also to the molecule that is released therefrom.

The term "hydrophobic drug" is herein defined as a drug with a clogP value (the calculated logarithm of its partition coefficient between n-octanol and water log(c_{octanol}/c_{water})) of >3. cLogP values may be estimated using CDD Vault software (Collaborative Drug Discovery Inc.) based on the method described in Gedeck et al., 2017, J. Chem. Inf. Model. 57, 8, 1847-1858. Model training information using publicly available datasets is available from CDD.

Aggregation is a process in which molecules self-associate with each other. In the context of the present invention, aggregation levels are assessed during the conjugation of a linker construct to an antibody. During conjugation, aggregation is quantified in terms of percentage monomer content. High percentage monomer values typically equate to a lower degree of aggregation. Percentage monomer values of 93% or 95% or higher typically mean negligible levels of aggregation or none, whereas values below this threshold typically translate to higher levels of aggregation.

### Description

The inventors have developed an antibody-drug conjugate, comprising a payload and a novel polyglycine linker system, which shows low to negligible aggregation propensity, in particular wherein the payload is a hydrophobic drug and the polyglycine linker system results in an ADC with increased hydrophilicity and as a consequence low to negligible propensity to aggregate. The resulting ADCs were found to display significant *in vivo* efficacy. It was also found that the resulting ADCs displayed improved conjugation efficiency.

The invention first and foremost concerns the antibody-drug conjugate. In a second aspect, the invention concerns a process for the synthesis of the antibody-drug conjugate according to the invention. In a third aspect, the invention concerns a linker-drug construct which is suitable to be used in the process according to the invention. In a fourth aspect, the invention concerns the medical use of the antibody-drug conjugate according to the invention, as well as a pharmaceutical composition comprising the antibody-drug conjugate according to the invention. The skilled person understands that all aspects are linked, and that everything said for the antibody-drug conjugate according to the invention equally applies to the process according to the invention, the linker-drug construct according to the invention, the uses according to the invention and the composition according to the invention, and vice versa.

The invention can be defined according to the following list of preferred embodiments:
The present invention provides an antibody-drug conjugate, having structure (1) 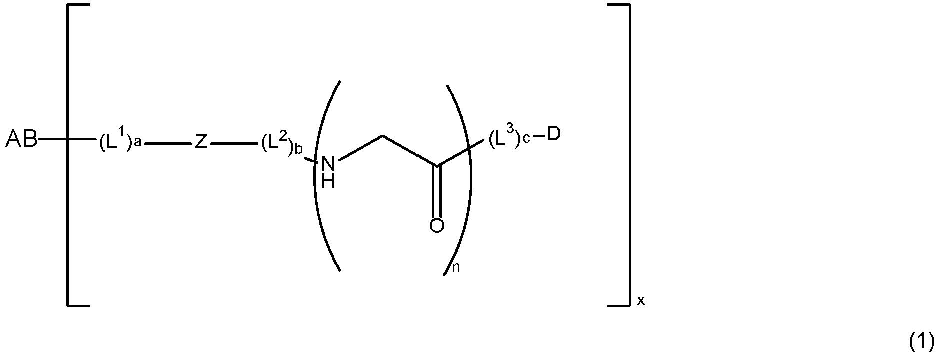 wherein:
AB is a cell binding agent such as an antibody;
n is an integer in the range of 5 - 8;
L¹ is a linker;
L² is a linker;
L³ is a linker;
a, b and c are independently 0 or 1;
Z is a connecting group obtained by a metal-free click reaction or by thiol ligation;
or Z is absent wherein a and b are 0 and the N terminus of the polyglycine is bonded directly to AB; D is a drug payload;
x is an integer in the range of 1 - 8.

In one embodiment, n is 5, 6 or 7. In a further embodiment, n is 5 or 6. In a yet further embodiment, n is 5.

In one embodiment, the present invention provides an antibody-drug conjugate of the present invention wherein L³ comprises a cathepsin cleavable peptide.

In one embodiment, the present invention provides an antibody-drug conjugate of the present invention wherein L³ is 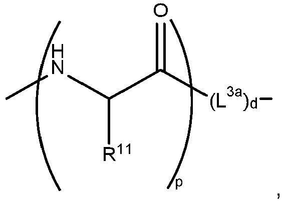 having structure (1a) 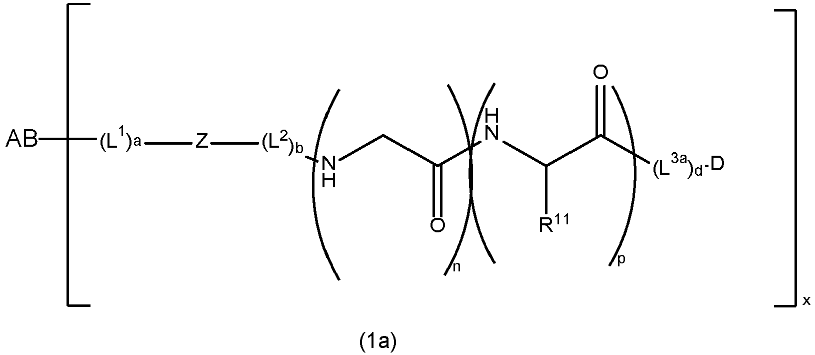 wherein:
AB is a cell binding agent such as an antibody;
n is an integer in the range of 5 - 8;
L¹ is a linker;
L² is a linker;
L^{3a} is a linker;
a, b and d are independently 0 or 1;
Z is a connecting group obtained by a metal-free click reaction or by thiol ligation;
or Z is absent wherein a and b are 0 and the N terminus of the polyglycine is conjugated directly to AB;
D is a drug payload;
p is an integer in the range 1 to 5;
each R¹¹ is independently an amino acid side chain;
x is an integer in the range of 1 - 8.

In one embodiment, the present invention provides an antibody-drug conjugate of the present invention, wherein each occurrence of the peptide (NH-CR¹¹-CO)p is selected from Val-Cit, Glu-Val-Cit, Val-Ala, Val-Lys, Val-Arg, Aclys-Val-Cit, Aclys-Val-Ala, Glu-Val-Ala, Asp-Val-Ala, Phe-Gly, Phe-Cit, Phe-Ala, Phe-Lys, Phe-Arg, Ala-Lys, Leu-Cit, Ile-Cit, Trp-Cit, Ala-Ala-Asn, Ala-Asn and Lys, preferably from Val-Cit, Glu-Val-Cit, Val-Ala, Glu-Val-Ala, Val-Lys, Phe-Gly. Phe-Cit, Phe-Ala, Phe-Lys, Ala-Ala-Asn, most preferably Val-Cit or Val-Ala.

In one embodiment, p is 2. In a further embodiment, p is 2 and n is 5.

In one embodiment, the present invention provides an antibody-drug conjugate of the present invention wherein L³ comprises a self-immolative group. In a further embodiment, L³ comprises an activatable self-immolative group.

In a further embodiment, the self-immolative group is a para-aminobenzyloxycarbonyl (PABC) derivative

The para-aminobenzyloxycarbonyl (PABC) derivative may be represented by general structure (L4): 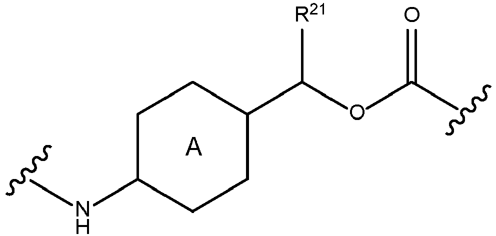 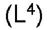 wherein;
A is an optionally substituted 5- or 6-membered aromatic or heteroaromatic ring;
R²¹ is selected from H, R²², C(O)OH and C(O)R²², wherein R²² is C₁ - C₂₄ (hetero)alkyl groups, C₃ - C₁₀ (hetero)cycloalkyl groups, C₂ - C₁₀ (hetero)aryl groups, C₃ - C₁₀ alkyl(hetero)aryl groups and C₃ - C₁₀ (hetero)arylalkyl groups, which are optionally substituted and optionally interrupted by one or more heteroatoms selected from O, S and NR²³ wherein R²³ is independently selected from the group consisting of hydrogen and C1 - C₄ alkyl groups.

In a further embodiment, the self-immolative group is an activatable self-immolative moiety and A is substituted by an activatable group (AG). In a yet further embodiment, AG is selected from 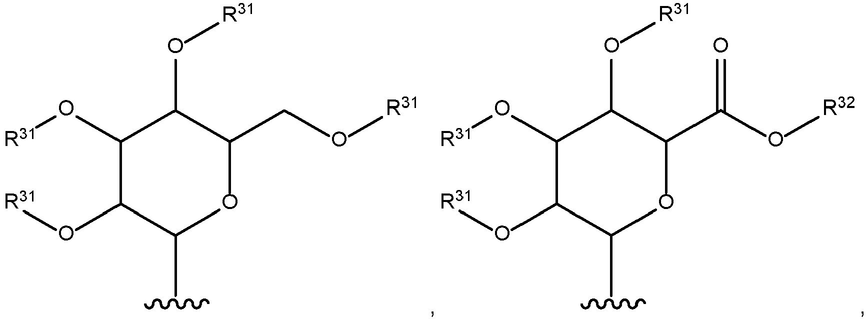 and 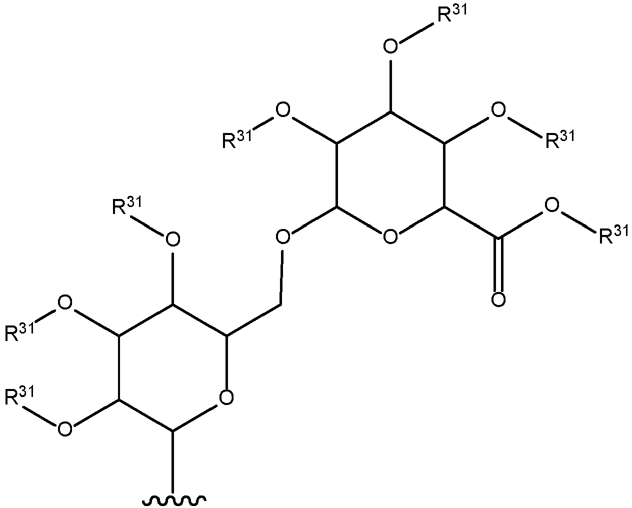 wherein
each R³¹ is independently selected from hydrogen and acetyl; and R³² is hydrogen or C₁-C₆ alkyl.

In one embodiment, A is optionally substituted phenyl, in particular optionally substituted 1,4-phenyl.

In one embodiment, the present invention provides an antibody-drug conjugate of the present invention having structure (1b) 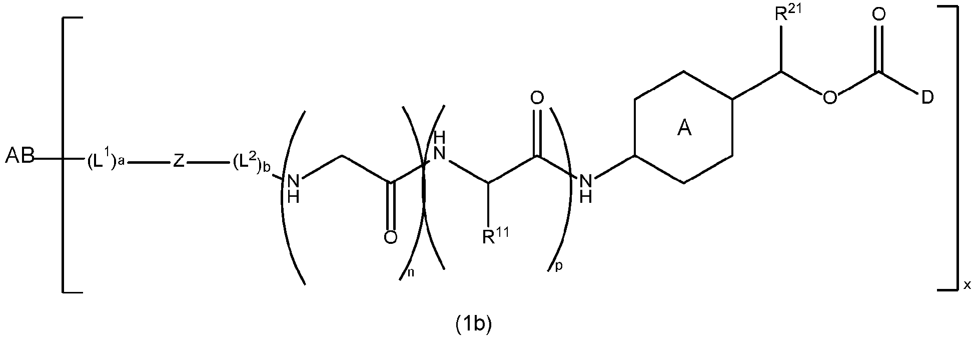 wherein:
AB is a cell binding agent, such as an antibody;
n is an integer in the range of 5 - 8;
L¹ is a linker;
L² is a linker;
L^{3a} is a linker;
a and b are independently 0 or 1;
Z is a connecting group obtained by a metal-free click reaction or by thiol ligation;
or Z is absent wherein a and b are 0 and the N terminus of the polyglycine is bonded directly to AB;
D is a drug payload;
p is an integer in the range 0 to 5;
each R¹¹ is independently an amino acid side chain;
A is an optionally substituted 5- or 6-membered aromatic or heteroaromatic ring;
R²¹ is selected from H, R²², C(O)OH and C(O)R²², wherein R²² is C₁ - C₂₄ (hetero)alkyl groups, C₃ - C₁₀ (hetero)cycloalkyl groups, C₂ - C₁₀ (hetero)aryl groups, C₃ - C₁₀ alkyl(hetero)aryl groups and C₃ - C₁₀ (hetero)arylalkyl groups, which are optionally substituted and optionally interrupted by one or more heteroatoms selected from O, S and NR²³ wherein R²³ is independently selected from the group consisting of hydrogen and C₁ - C₄ alkyl groups.
x is an integer in the range of 1 - 8.

In one embodiment, wherein a= 0 and the conjugation site is a cysteine residue, particularly the cysteine residue is naturally present in the antibody, optionally after reduction of a disulfide bond, or wherein the cysteine residue is engineered, particularly by substitution of an amino acid by cysteine, cysteine insertion or introduction of a single cysteine residue or a peptide fragment containing a cysteine residue at the N- or C-terminus.

In one embodiment, the antibody-drug conjugate has structure (1c) 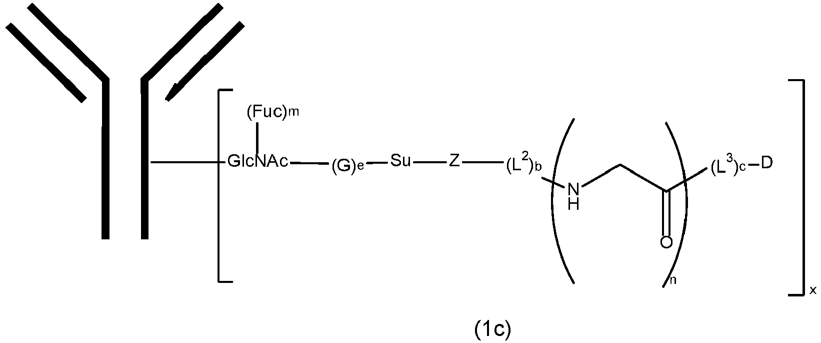
z, L², L³, D, b, c, n and x are as defined herein;
e is an integer in the range of 0 - 20;
Su is a monosaccharide;
G is a monosaccharide moiety;
GlcNAc is an N-acetylglucosamine moiety;
Fuc is a fucose moiety;
m is 0 or 1.

The antibody-drug conjugate according to any one of the preceding embodiments, wherein the drug payload is a hydrophobic drug.

The antibody-drug conjugate according to any one of the preceding embodiments, wherein the drug payload iscamptothecin or a derivative thereof such as exatecan..or deruxtecan, in particular exatecan.

In another aspect, the present invention provides a process for the synthesis of the antibody-drug conjugate of the present invention, comprising reacting
(i) a modified antibody of structure AB-((L¹)ₐ-F)x, wherein:
   AB is an antibody;
   L¹ is a linker;
   a is 0 or 1;
   F is a click probe capable of reacting with Q in a metal-free click reaction or a thiol or precursor thereof
   x is an integer in the range of 1 - 8;
   with
(ii) a linker-drug construct according to structure (5): 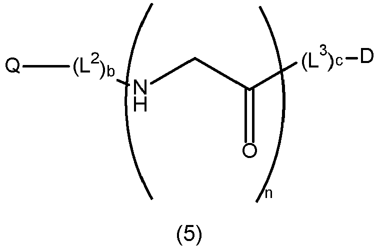 wherein:
   Q is a click probe capable of performing a metal-free click reaction or a thiol-reactive probe;
   L²,L³,D, n, b and c are as defined herein;
to form an antibody-drug conjugate wherein the drug is covalently attached to the antibody via connecting group Z that is formed by a metal-free click reaction between Q and F or by thiol ligation between Q and F.

In one embodiment, the click probe Q comprises a cyclic alkyne moiety, a cyclic alkene moiety or a tetrazine moiety; or the thiol-reactive probe Q comprises a maleimide moiety.

In one embodiment, the click probe Q is selected from the group consisting of, optionally substituted, (hetero)cyclopropenyl group, (hetero)cyclobutenyl group, trans-(hetero)cycloheptenyl group, trans-(hetero)cyclooctenyl group, trans-(hetero)cyclononenyl group or trans-(hetero)cyclodecynyl group.

In one embodiment, the click probe F is selected from the group consisting of azide, tetrazine, triazine, nitrone, nitrile oxide, nitrile imine, diazo compound, ortho-quinone, dioxothiophene, sydnone and a cyclic alkene moiety, preferably click probe F is an azide moiety.

In one embodiment, the process of the present invention, wherein the click reaction is a 1,3- dipolar cycloaddition or a (4+2) cycloaddition.

In one embodiment, F is a thiol.

In one embodiment, the thiol ligation is a nucleophilic reaction, preferably a Michael addition or a nucleophilic substitution.

In one embodiment, wherein a = 0 and the conjugation site is a cysteine residue, particularly the cysteine residue is naturally present in the antibody, optionally after reduction of a disulfide bond, or wherein the cysteine residue is engineered, preferably by substitution of an amino acid by cysteine, cysteine insertion or introduction of a single cysteine residue or a peptide fragment containing a cysteine residue at the N- or C-terminus
In one embodiment, :the click probe F is selected from the group consisting of an azide, tetrazine, triazine, nitrone, nitrile oxide, nitrile imine, diazo compound, ortho-quinone, dioxothiophene, sydnone and cyclic alkene moiety, particularly click probe F is an azide moiety; and/or wherein the click reaction is a 1,3- dipolar cycloaddition or a (4+2) cycloaddition; or F is a thiol; and/or wherein the thiol ligation is a Michael addition or a nucleophilic substitution.

In one embodiment, the process of the invention , wherein AB-((L¹)a-F)x is according to structure (6): 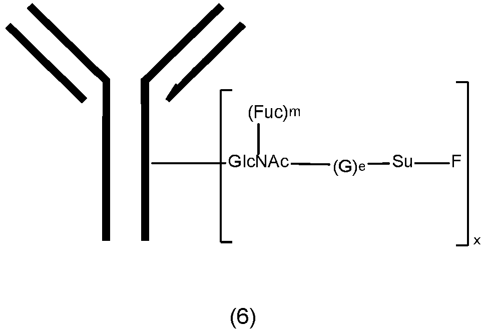 wherein:
e is an integer in the range of 0- 10;
Su is a monosaccharide;
G is a monosaccharide moiety;
GlcNAc is an N-acetylglucosamine moiety;
Fuc is a fucose moiety;
m is 0 or 1.

In another aspect, the present invention provides a linker-drug construct according to structure (5): 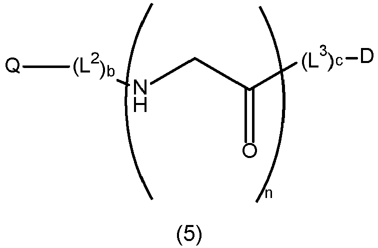 wherein:
Q is a click probe capable of performing a metal-free click reaction or a thiol-reactive probe;
AB is an antibody;
n is an integer in the range of 5 - 8;
L² is a linker;
L³ is a linker;
b and c are independently 0 or 1;
L²,L³,D, n, b and c are as defined herein.

In one embodiment, the click probe Q comprises a cyclic alkyne moiety or a cyclic alkene moiety, or wherein the thiol-reactive probe Q comprises a maleimide moiety.

In one embodiment, the click probe Q comprises a cyclic alkyne moiety or a cyclic alkene moiety
In one embodiment, the the click probe Q comprises a tetrazine moiety
In one embodiment, the click probe Q is selected from the group consisting of, optionally substituted, (hetero)cyclopropenyl group, (hetero)cyclobutenyl group, trans-(hetero)cycloheptenyl group, trans-(hetero)cyclooctenyl group, trans-(hetero)cyclononenyl group or trans-(hetero)cyclodecynyl group.

In another aspect, the present invention provides a pharmaceutical composition comprising the antibody-drug conjugate of the present invention and a pharmaceutically acceptable carrier.

In another aspect, the present invention provides the antibody-drug conjugate of the present invention, for use in the treatment of a subject in need thereof.

In another aspect, the present invention provides the antibody-drug conjugate of the present invention, for use in the treatment of cancer.

In another aspect, the present invention provides the antibody-drug conjugate for use according to the invention, wherein the cancer is a HER2-positive cancer, preferably breast, stomach, colon, lung, pancreatic, urothelial, brain or ovarian cancer.

### The antibody-drug conjugate

The antibody-drug conjugate according to the invention has structure **(1):** 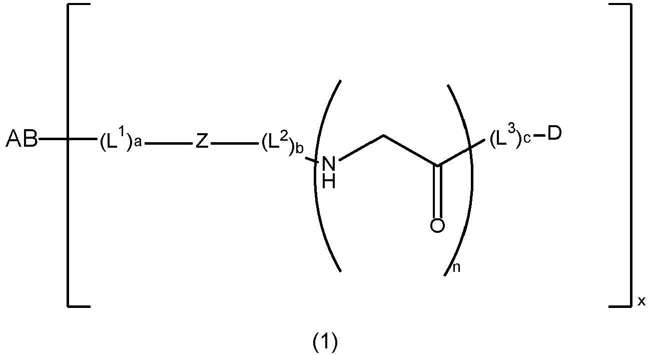 wherein:
AB is a cell binding agent, such as an antibody;
n is an integer in the range of 5 - 8;
L¹ is a linker;
L² is a linker;
L³ is a linker;
a, b and c are independently 0 or 1;
Z is a connecting group obtained by a metal-free click reaction or by thiol ligation;
or Z is absent wherein a and b are 0 and the N terminus of the polyglycine is bonded directly to AB; D is a drug payload;
x is an integer in the range of 1 - 8.

The conjugate according to the invention comprises a polyglycine (Gly)n linker wherein n is an integer in the range 5 -8. The conjugate according to the invention may comprise a self-immolative group and/or cleavable linker, in particular comprising a peptide spacer and/or a para aminobenzyloxycarbonyl (PABC) moiety or derivative hereof
In particular, n is 5, 6 or 7, more particularly, n is 5 or 6, most particularly, n is 5.

In particular, the antibody-drug conjugate of the present invention comprises a cathepsin cleavable peptide.

The peptide spacer is defined by (NH-CR¹¹-CO)p, wherein R¹¹ represents an amino acid
side chain as known in the art. Herein, the amino acid may be a natural or a synthetic amino acid. Particularly, the amino acid(s) are all in their L-configuration. p is an integer in the range of 1 - 5, particularly in the range of 2 - 5. Thus, the peptide spacer contains 1 - 5 amino acids. Particularly, the peptide is a dipeptide (p = 2) or tripeptide (p = 3), most preferably the peptide spacer is a dipeptide. Although any peptide spacer may be used, particularly the peptide spacer is selected from Val-Cit, Glu-Val-Cit, Val-Ala, Val-Lys, Val-Arg, AcLys-Val-Cit, AcLys-Val-Ala, Glu-Val-Ala, Asp-Val-Ala, Phe-Gly, Phe-Cit, Phe-Ala, Phe-Lys, Phe-Arg, Ala-Lys, Leu-Cit, Ile-Cit, Trp-Cit, Ala-Ala-Asn, Ala-Asn and Lys, preferably from Val-Cit, Glu-Val-Cit,Val-Ala, Glu-Val-Ala, Val-Lys, Phe-Gly. Phe-Cit, Phe-Ala, Phe-Lys, Ala-Ala-Asn, most preferably Val-Cit or Val-Ala. In one embodiment, the peptide spacer is Val-Cit. In another embodiment, the peptide spacer is Val-Ala.

In one embodiment, the peptide spacer and the polyglycine linker (Gly)ₙ are adjacent and form an uninterrupted polypeptide. In one embodiment, this uninterrupted polypeptide comprises the peptide sequence Gly-Gly-Phe-Gly (GGFG) at its C or N terminus.

In one embodiment L³ has the structure L^{3'} 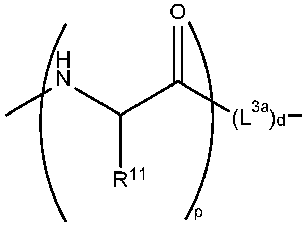 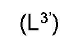 wherein(L^{3'})
R¹¹ represents the amino acid side chain;
L^{3a} is a linker;
d is 0 or 1;
p is an integer in the range 1 to 5.

In particular, R¹¹ is selected from the side chains of alanine, cysteine, aspartic acid, glutamic acid, phenylalanine, glycine, histidine, isoleucine, lysine, acetyllysine, leucine, methionine, asparagine, pyrrolysine, proline, glutamine, arginine, serine, threonine, selenocysteine, valine, tryptophan, tyrosine and citrulline. Particular amino acid side chains are those of Val, Cit, Ala, Lys, Arg, AcLys, Phe, Leu, Ile, Trp, Glu, Asp and Asn, more preferably from the side chains of Val, Cit, Ala, Glu and Lys. Alternatively worded, R¹¹ is preferably selected from CH₃ (Ala), CH₂CH(CH₃)₂ (Leu), CH₂CH₂CH₂NHC(O)NH₂ (Cit), CH₂CH₂CH₂CH₂NH₂(Lys), CH₂CH₂CH₂NHC(O)CH₃ (AcLys), CH₂CH₂CH₂NHC(=NH)NH₂ (Arg), CH₂Ph (Phe), CH(CH₃)₂ (Val), CH(CH₃)CH₂CH₃ (Ile), CH₂C(O)NH₂ (Asn), CH₂CH₂C(O)OH (Glu), CH₂C(O)OH (Asp) and CH₂(1H-indol-3-yl) (Trp). Most particular embodiments of R¹¹ are CH₃ (Ala), CH₂CH₂CH₂NHC(O)NH₂ (Cit), CH₂CH₂CH₂CH₂NH₂ (Lys), CH₂CH₂C(O)OH (Glu) and CH(CH₃)₂ (Val). Most particularly, R¹¹ is CH₃ (Ala), CH₂CH₂CH₂NHC(O)NH₂ (Cit), CH₂CH₂CH₂CH₂NH₂ (Lys),orCH(CH₃)₂ (Val).

In particular, the peptide spacer may be represented by general structure (L^{3b}): 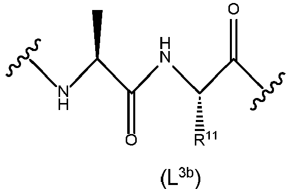 wherein, R¹¹ is as defined above, particularly R¹¹ is CH₃ (Ala) or CH₂CH₂CH₂NHC(O)NH₂ (Cit).

The wavy lines represent the connection to the remainder of the conjugate.

In particular, p is 2, more particularly, p is 2 and n is 5.

In one embodiment, the present invention provides an antibody-drug conjugate of the present invention having structure (1a) 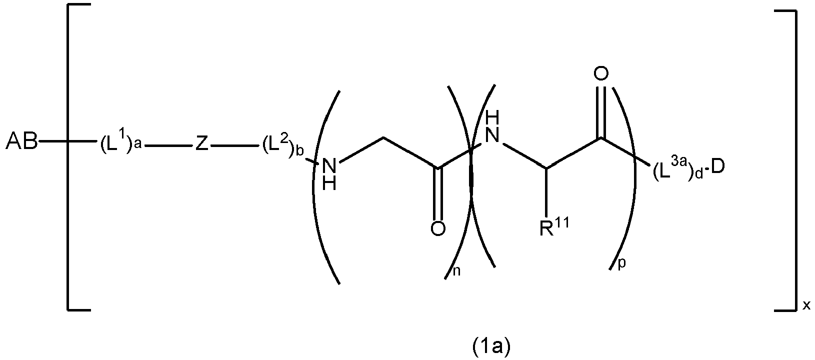 wherein:
AB is a cell binding agent such as an antibody;
n is an integer in the range of 5 - 8;
L¹ is a linker;
L² is a linker;
L^{3a} is a linker;
a, b and d are independently 0 or 1;
Z is a connecting group obtained by a metal-free click reaction or by thiol ligation;
or Z is absent wherein a and b are 0 and the N terminus of the polyglycine is conjugated directly to AB;
D is a drug payload;
p is an integer in the range 1 to 5;
each R¹¹ is independently an amino acid side chain;
x is an integer in the range of 1 - 8.

An antibody-drug conjugate of the present invention wherein L³ may comprise a self-immolative group, in particular an activatable self-immolative group.

Particularly, the self-immolative group is a para-aminobenzyloxycarbonyl (PABC) derivative
The para-aminobenzyloxycarbonyl (PABC) derivative may be represented by general structure (L⁴): 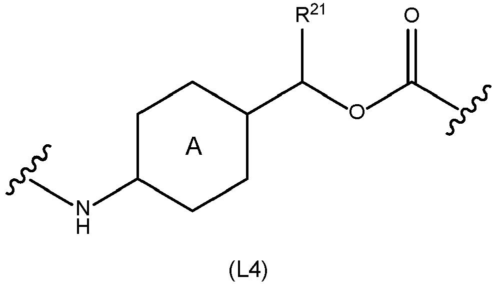 wherein;
A is an optionally substituted 5- or 6-membered aromatic or heteroaromatic ring;
R²¹ is selected from H, R²², C(O)OH and C(O)R²², wherein R²² is C₁ - C₂₄ (hetero)alkyl groups, C₃ - C₁₀ (hetero)cycloalkyl groups, C₂ - C₁₀ (hetero)aryl groups, C₃ - C₁₀ alkyl(hetero)aryl groups and C₃ - C₁₀ (hetero)arylalkyl groups, which are optionally substituted and optionally interrupted by one or more heteroatoms selected from O, S and NR²³ wherein R²³ is independently selected from the group consisting of hydrogen and C₁ - C₄ alkyl groups.
The wavy lines represent the connection to the remainder of the conjugate

In particular, the self-immolative group is an activatable self-immolative moiety and A is substituted by an activatable group (AG). More particularly, AG is selected from 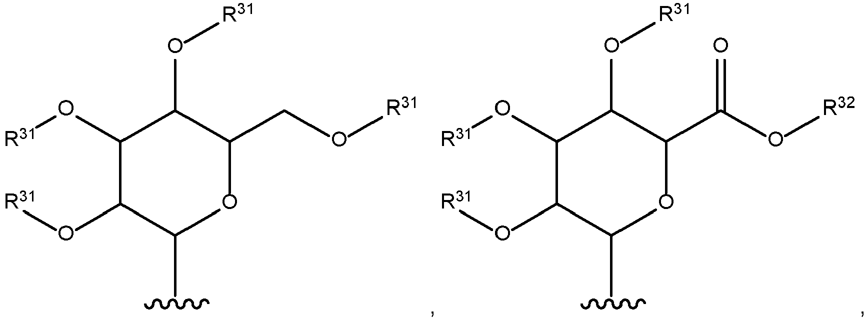 and 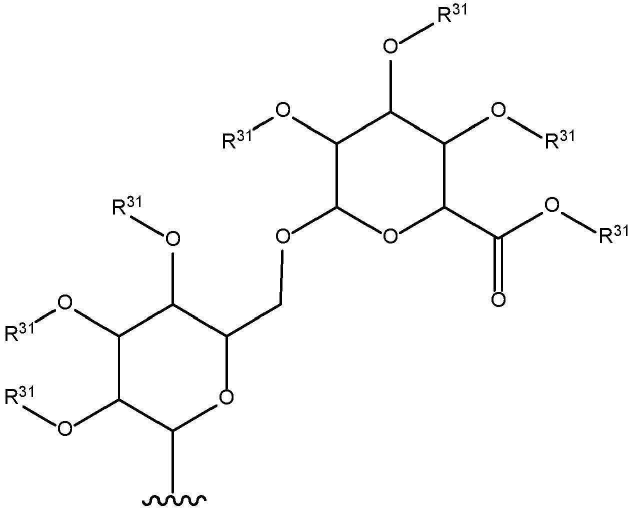 wherein
each.R³¹ is independently selected from hydrogen and acetyl; and R³² is hydrogen or C₁-C₆ alkyl. The wavy line represents the connection to A.

In particular, antibody-drug conjugate of the present invention having structure (1b) 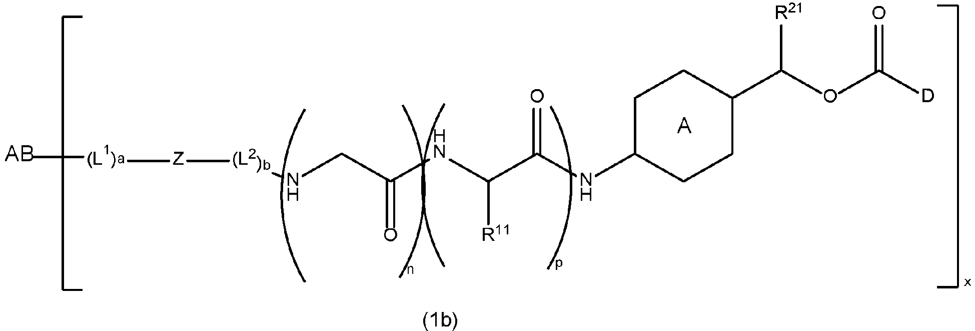 wherein:
AB is an antibody;
n is an integer in the range of 5 - 8;
L¹ is a linker;
L² is a linker;
L^{3a} is a linker;
a and b are independently 0 or 1;
Z is a connecting group obtained by a metal-free click reaction or by thiol ligation;
or Z is absent wherein a and b are 0 and the N terminus of the polyglycine is bonded directly to AB; D is a drug payload;
p is an integer in the range 0 to 5;
each R¹¹ is independently an amino acid side chain;
A is an optionally substituted 5- or 6-membered aromatic or heteroaromatic ring;
R²¹ is selected from H, R²², C(O)OH and C(O)R²², wherein R²² is C₁ - C₂₄ (hetero)alkyl groups, C₃ - C₁₀ (hetero)cycloalkyl groups, C₂ - C₁₀ (hetero)aryl groups, C₃ - C₁₀ alkyl(hetero)aryl groups and C₃ - C₁₀ (hetero)arylalkyl groups, which are optionally substituted and optionally interrupted by one or more heteroatoms selected from O, S and NR²³ wherein R²³ is independently selected from the group consisting of hydrogen and C₁ - C₄ alkyl groups.
x is an integer in the range of 1 - 8.
Suitable 5-membered rings are oxazole, thiazole and furan. Suitable 6-membered rings are phenyl and pyridyl. In particular, A is optionally substituted 1,4-phenyl, 2,5-pyridyl or 3,6-pyridyl. Most particularly, A is 1,4-phenyl.

In particular, R²¹ is H or C(O)R²², wherein R²² = 4-methyl-piperazine or morpholine. In particular, R²¹ is H

### Linkers (L¹, L², L³ and L3^{a})

Linkers, also referred to as linking units, are well known in the art and any suitable linker may be used. In the context of the present invention, the payload is chemically connected to the polyglycine linker (Gly)ₙ via linker L³; the polyglycine linker (Gly)n is chemically connected to the connecting group Z, obtained by a metal-free click reaction or thiol ligation, via linker L² and the connecting group Z is chemically connected to the antibody via linker L¹. A linker, especially linker L², may contain one or more branch-points for attachment of multiple payloads to a single connecting group.

Linker L¹ may be present (a = 1) or absent (a = 0). In the case wherein linker L¹ is absent, click probe F is attached directly to the antibody.

Typically, a = 0 in the case wherein the conjugation is via thiol ligation. Typically, a = 1 in the case wherein the conjugation is via a click reaction.

In the case wherein, Z is absent, a=0 and b=0, the N terminus of the polyglycine linker (Gly)ₙ is bonded directly to the antibody, for example via a microbial transglutaminase (MTGase) conjugation.

A linker may for example be selected from the group consisting of linear or branched C₁- C₂₀₀ alkylene groups, C₂-C₂₀₀ alkenylene groups, C₂-C₂₀₀ alkynylene groups, C₃-C₂₀₀ cycloalkylene groups, C₅-C₂₀₀ cycloalkenylene groups, C₈-C₂₀₀ cycloalkynylene groups, C₇-C₂₀₀ alkylarylene groups, C₇-C₂₀₀ arylalkylene groups, C₈-C₂₀₀ arylalkenylene groups, C₉-C₂₀₀ arylalkynylene groups. Optionally the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups may be substituted, and optionally said groups may be interrupted by one or more heteroatoms, preferably 1 to 100 heteroatoms, said heteroatoms preferably being selected from the group consisting of O, S(O)y and NR¹², wherein y is 0, 1 or 2,preferably y = 2, and R¹² is independently selected from the group consisting of hydrogen, halogen, C₁ - C₂₄ alkyl groups, C₈ - C₂₄ (hetero)aryl groups, C₁ - C₂₄ alkyl(hetero)aryl groups and C₁ - C₂₄ (hetero)arylalkyl groups.

In particular, L² is selected from -(CR¹R²)y-, -(CR¹R²)y-C(O)-, and -C(O)-(CR¹R²)y-C(O)-, wherein y is an integer in the range 0 to 6, wherein one or more CR¹R² may be optionally replaced by NR¹; R¹ and R² are independently selected from H, R^{2a}, C(O)OH and C(O)R^{2a}, wherein R^{2a} is selected from optionally substituted C₁ - C₆ (hetero)alkyl groups, C₃ - C₁₀ (hetero)cycloalkyl groups, C₆ - C₁₀ (hetero)aryl groups, C₇ - C₁₄ alkyl(hetero)aryl groups and C₇ - C₁₄ (hetero)arylalkyl groups; wherein R^{3a} is independently selected from hydrogen and C₁ - C₄ alkyl groups.

In particular, R¹ and R² are H.

In one embodiment, L² is -(CH₂)₁₋₅-C(O)-. In a particular embodiment L² is -CH₂C(O)- or - CH₂CH₂C(O)-.

### Branching moiety

In one embodiment, the linker of the conjugate according to the invention contains a branching moiety. A "branching moiety" in the context of the present invention refers to a moiety that is embedded in a linker connecting three moieties. In other words, the branching moiety comprises at least three bonds to other moieties, typically one bond to antibody AB, connecting to Z, one bond to the payload and one bond to a second payload. The branching moiety is preferably embedded in linker L². Any moiety that contains at least three bonds to other moieties is suitable as branching moiety in the context of the present invention. In a preferred embodiment, the branching moiety BM is selected from a carbon atom, a nitrogen atom, a phosphorus atom, a (hetero)aromatic ring, a (hetero)cycle or a polycyclic moiety. Most preferably, the branching moiety is a nitrogen atom.

In one embodiment, linker L² contains a branching moiety, in particular via a nitrogen atom,
such that two payloads are connected to a single moiety Z.

In a particular embodiment, L² has structure (2): 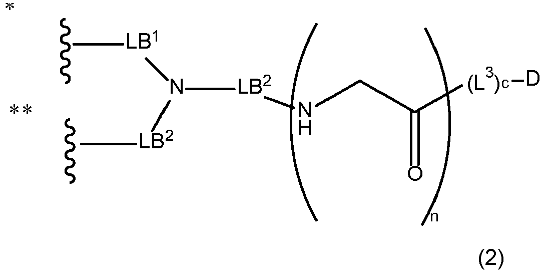 wherein, the wavy bond labelled with* is connected to Z and the wavy bond labeled with ** is connected to the N terminal NH of the polyglycine linker. LB¹ and LB² are each individually linker moieties, The two linker-payload moieties connected to the branching nitrogen atom may be the same or different. In particular, each occurrence of D is the same. In a particular embodiment, each occurrence of LB² is the same. In a particular embodiment, each occurrence of n is the same. In particular, each occurrence of L³ and c is the same.

### Connecting group Z

Z is a connecting group. The term "connecting group" refers to a structural element connecting one part of the conjugate and another part of the same bioconjugate. In (1), Z connects antibody AB with the payload, via a linker. Connecting group Z is a moiety is obtainable by a metal-free click reaction or by thiol ligation. As the skilled person understands, the exact nature of Z depends on the nature of F and Q. Preferred embodiments for Q and F are defined further below.

In a first embodiment, connecting group Z is obtainable by a metal-free click reaction. Herein, connecting group Z may contain a triazole moiety, an isoxazole moiety, a dihydroisoxazole moiety, a bicyclo[2.2.2]octa-5,7-diene-2,3-dione moiety, a bicyclo[2.2.2]octa-5- ene-2,3-dione moiety, a 7-thiabicyclo[2.2.1]hepta-2,5-diene-7,7-dioxide moiety, a 7- thiabicyclo[2.2.1]hept-2-ene- 7,7-dioxide moiety, a pyrazole moiety, a pyridine moiety, a dihydropyridine moiety, a pyridazine moiety or a dihydropyridazine moiety

In a particular embodiment, Q contains a cyclic alkyne moiety and F is an azide, and Z contains a triazole moiety that is formed by 1,3-dipolar cycloaddition of the alkyne moiety with the azide moiety In a second particular embodiment, connecting group Z is obtainable by thiol ligation wherein, connecting group Z has one connection to L2 and at least one connection to AB, possibly via L1. One connection group Z may also have two connections to AB, wherein a cross-linking thiol-reactive probe Q is used. In one embodiment, L1 is absent (a=0) and Z is connected directly to AB. In a particular embodiment, connection group Z comprises a succinimidyl ring or its ring- opened succinic acid amide derivative

### Mode of conjugation

The conjugates according to the invention may be prepared by a mode of conjugation wherein the conjugation reaction is a thiol ligation or a metal-free click reaction. Alternatively, in the case wherein Z is absent, a=0 and b=0, the N terminus of the polyglycine linker (Gly)n is bonded directly to the antibody, for example via a microbial transglutaminase (MTGase) conjugation.

The nature of the modified antibody AB-((L1)a-F)x, in particular the nature of L1, a and x, depends on the mode of conjugation employed. x refers to the number of attachment points (conjugation sites) of (L1)a on the antibody and is an integer in the range of 1 - 8. In case wherein Z (in the conjugate) or F (in the modified antibody) is directly attached to the antibody, L1 is absent and a = 0. In this embodiment, the modified antibody may also be referred to as AB-(F)x.

x represents the amount of probes F present on the antibody, optionally via linker L1, and is an integer in the range of 1 - 8. Particularly, x is an integer in the range of 1 - 6, more particularly x = 1, 2, 3 or 4, even more particularly x = 1 or 2, most particularly x = 2. Typically, x refers to the average number of moieties Z connected to the antibody. In a particular embodiment, especially in case the conjugation is by click reaction, the conjugate according to the invention normally has the same amount of moieties Z connected to the antibody, although the conjugation reaction may at times be slightly incomplete. Notably, each linker L2 may contain more than one payload, such as 1 or 2 payload molecules per linker L2.

In a particular embodiment, a = 1 and the mode of conjugation involves conjugation via the glycan of the antibody, preferably via an N-glycosylation site. Typically, the modified antibody comprises one or more glycans with structure -GIcNAc(Fuc)m-(G)e-Su-F, wherein e is an integer in the range of 0 - 20; Su is a monosaccharide; G is a monosaccharide moiety; GlcNAc is an N- acetylglucosamine moiety; Fuc is a fucose moiety; and m is 0 or 1. In other words, L1 is particularly represented by - GlcNAc(Fuc)m-(G)e-Su- wherein GlcNAc(Fuc)m is attached to the peptide part of the antibody and Su is attached to Z or F, GlcNAc is the core N-acetylglucosamine moiety which is typically present in the glycan structure of antibodies. The core N-acetylglucosamine moiety refers to the N-acetylglucosamine moiety that is directly attached to the peptide chain of the antibody. This core N-acetylglucosamine moiety is optionally fucosylated (m is 0 or 1), which is a common feature of antibodies.

(G)e represents the glycoform of the antibody. The present invention may be applied to antibodies of any glycoform. Typical monosaccharides that are present in glycans, and from among which G may be selected, include glucose, galactose, mannose, fucose, N-acetylglucosamine, N-acetylgalactosamine, N-acetylneuraminic acid and xylose. (G)e may thus be a linear or branched oligosaccharide, comprising e monosaccharide moieties. Typical glycans have e in the range of 4-16, preferably 6-10. In a preferred embodiment, the antibody is trimmed and e = 0. Such trimming may be performed by an endoglycosidase enzyme such as EndoS. Conjugation via the glycan preferably employs an N-glycosylation site, more preferably an N-glycosylation site connected to an asparagine amino acid of the antibody, most preferably to the conserved glycosylation site at amino acid N297 of the antibody.

Su is a monosaccharide containing F. Su is preferably selected from the group consisting of galactose (Gal), mannose (Man), glucose (Glc), N-acetylglucosamine (GIcNAc), N-acetylgalactosamine (GaINAc), N-acetylneuraminic acid or sialic acid (Sial) and fucose (Fuc). Su is preferably a glucose or galactose derivative, more preferably a galactose derivative, most preferably N-acetylgalactosamine.

In an alternative embodiment, a = 0 and the conjugation site at the antibody is a cysteine residue, particularly wherein the cysteine residue is naturally present in the antibody,
optionally after reduction of a disulfide bond as known in the art, or wherein the cysteine residue is engineered, preferably by substitution of an amino acid by cysteine, cysteine insertion or introduction of a single cysteine residue or a peptide fragment containing a cysteine residue at the N- or C-terminus.

In the alternative embodiment wherein Z is absent, a=0 and b=0, the N terminus of the polyglycine linker (Gly)n is bonded directly to the antibody; L1 and L2 are therefore absent if Z is absent. If Z is absent, the N terminus of the polyglycine linker (Gly)n is therefore bonded directly to the antibody in the absence of any linker, for example in the absence of any linker comprising a sortase recognition sequence motif.LPTXG (X is any amino acid).

In one embodiment, Z is absent, a=0 , b=0, and the N terminus of the polyglycine linker (Gly)n is conjugated directly to the antibody via a microbial transglutaminase (MTGase), Microbial transglutaminase (mTG) catalyzes a transamidation reaction between certain surface-exposed glutamine (Gln) and lysine (Lys) residues on protein substrates, crosslinking the two via an isopeptide bond. The catalytic mechanism of mTG involves a nucleophilic attack of the target acyl donor, Gln, by the enzyme's active site cysteine to form a thioester intermediate. Subsequent nucleophilic attack of the thioester by an amine substrate serving as the acyl acceptor results in the formation of the transamidated product. In particular, the conjugation site is Q295.

The most commonly used mTGase is the 38-kDa enzyme derived from Streptomyces mobaraensis. Another transglutaminase found in bacteria is from Bacillus subtilisin (See US5,731,183). Various variants of mTGase have been developed such as the mTGases disclosed in WO2017/059158 and WO2017/059160 (Merck) which have for example an amino acid substitution selected from the group consisting of (A) E300A, (B) I240A and P241A, (C) E249Q, and (D) E300A and Y302A.

AB represents the antibody. The skilled person understands that the present invention can be applied to any antibody.

### Process for the synthesis of the antibody-drug conjugate

In a second aspect, the invention concerns a process for preparing the antibody-drug conjugate according to the invention. A process according to the invention comprises reacting (i) a modified antibody of structure AB-((L1)a-F)x with (ii) a linker-drug construct according to structure (5). The reaction is a conjugation reaction, which forms a covalent attachment between the payload and the antibody. The reaction is a metal-free click reaction or a thiol ligation, and forms an antibody-drug conjugate wherein the drug is covalently attached to the antibody via connecting group Z that is formed by a metal-free click reaction between Q and F or a thiol ligation between Q and F. Alternatively, in the case wherein, a=0 and b=0 and F is absent, the N terminus of the polyglycine linker (Gly)n may be bonded directly to the antibody, for example via a microbial transglutaminase (MTGase) conjugation. In the process according to the invention, the modified antibody of structure AB-((L1)a-F)x, wherein AB is an antibody, L1 is a linker, a is 0 or 1, F is a click probe capable of reacting with Q in a metal-free click reaction or a thiol or precursor thereof capable of reacting with Q in a thiol ligation and x is an integer in the range of 1 - 8. Upon reacting F with Q of the linker-drug construct according to structure (5) via a metal-free click reaction or a thiol ligation, connecting group Z is formed. In one embodiment, the modified antibody may be referred to as AB-(F)x. Methods of preparing modified antibody are known in the art, e.g. from WO2014/065661, WO2016/170186 and WO2016/053107, which are incorporated herein by reference. From the same documents, the conjugation reaction between the modified glycoprotein and a linker-drug construct comprising a cytotoxin and a click probe is known to the skilled person.

In the process according to the invention, the linker-drug construct has structure (5): 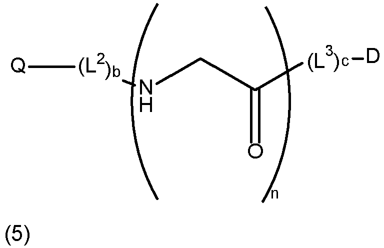 wherein:
Q is a click probe capable of performing a metal-free click reaction or a thiol-reactive probe;
L2,L3,D, n, b and c are as defined herein above

### Metal-free click reaction

Metal-free click reactions are well-known in the art (see e.g. from WO2014/065661 and Nguyen and Prescher, Nature rev. 2020, doi: 10.1038/s41570-020-0205-0, both incorporated by reference) and may typically take the form of a 1,3-dipolar cycloaddition or (4+2) cycloaddition. The alkyneazidecycloaddition may be strain-promoted (e.g. a strain-promoted alkyne-azide cycloaddition, SPAAC). In a particular embodiment, the bioconjugation reaction is a metal-free strain -promoted cycloaddition, most particularly metal-free strain-promoted alkyne-azide cycloaddition. In a particular embodiment, conjugation is accomplished via a cycloaddition, such as a (4+2) cycloaddition or a 1,3-dipolar cycloaddition, preferably the 1,3-dipolar cycloaddition. A typical (4+2) cycloaddition is the Diels-Alder reaction, wherein Q is a diene or a dienophile. As appreciated by the skilled person, the term "diene" in the context of the Diels-Alder reaction refers to 1,3-(hetero)dienes, and includes conjugated dienes (R2C=CR-CR=CR2), imines (e.g. R2C=CR-N=CR2 or R2C=CR-CR=NR, R2C=N-N=CR2) and carbonyls (e.g. R2C=CR-CR=O or O=CR-CR=O). Hetero-Diels-Alder reactions with N- and O-containing dienes are known in the art. Any diene known in the art to be suitable for (4+2) cycloadditions may be used as reactive group Q. Particular dienes include tetrazines, 1,2-quinones and triazines. Although any dienophile known in the art to be suitable for (4+2) cycloadditions may be used as reactive group Q, the dienophile is particularly an alkene or alkyne group as described above, most particularly an alkyne group or cyclic alkene. For conjugation via a (4+2) cycloaddition, Q may be a dienophile (and F is a diene), more particularly Q is or comprises an alkynyl group.. Alternatively, for conjugation via a (4+2) cycloaddition, Q may be a diene (and F is a dienophile), more particularly Q is or comprises a tetrazine group
For a 1,3-dipolar cycloaddition, Q is a 1,3-dipole or a dipolarophile. Any 1,3-dipole known in the art to be suitable for 1,3-dipolar cycloadditions may be used as reactive group Q. Particular 1,3-dipoles include azido groups, nitrone groups, nitrile oxide groups, nitrile imine groups and diazo groups. Although any dipolarophile known in the art to be suitable for 1,3-dipolar cycloadditions may be used as reactive groups Q, the dipolarophile is particularly an alkene or alkyne group, most particularly an alkyne group. For conjugation via a 1,3-dipolar cycloaddition, in particular Q is a dipolarophile (and F is a 1,3-dipole), more particularly Q is or comprises an alkynyl group.

Thus, in a particular embodiment, Q is selected from dipolarophiles and dienophiles.

### Click probe Q

Click probe Q is used in the conjugation reaction to connect the linker-drug construct to the antibody of structure AB-((L1)a-F)x. Q is reactive towards click probe F in a metal-free click reaction. Such click probes are known in the art and include cyclic alkene, cyclic alkyne, azide, tetrazine, triazine, nitrone, nitrile oxide, nitrile imine, diazo compound, ortho-quinone, dioxothiophene and sydnone. In particular, Q is a cyclic alkene or a cyclic alkyne moiety, most particularly Q is a cyclic alkyne moiety.

In a particular embodiment, the click probe Q comprises a cyclic alkyne moiety. The alkynyl group may also be referred to as a (hetero)cycloalkynyl group, i.e. a heterocycloalkynyl group or a cycloalkynyl group, wherein the (hetero)cycloalkynyl group is optionally substituted. Particularly, the (hetero)cycloalkynyl group is a (hetero)cycloheptynyl group, a (hetero)cyclooctynyl group, a (hetero)cyclononynyl group or a (hetero)cyclodecynyl group. Herein, the (hetero)cycloalkynes may optionally be substituted. Particularly, the (hetero)cycloalkynyl group is an optionally substituted (hetero)cycloheptynyl group or an optionally substituted (hetero)cyclooctynyl group. Most particularly, the (hetero)cycloalkynyl group is a (hetero)cyclooctynyl group, wherein the (hetero)cyclooctynyl group is optionally substituted.

Particular cycloalkynyl groups include a bicyclo[6.1.0]non-4-yn-9-yl] group (BCN group), which is optionally substituted or a 2-azatricyclo[10.4.0.04,9]hexadeca-1(16),4,6,8,12,14-hexaen-10-yn-2-yl] (DBCO group) which is optionally substituted.

### Thiol ligation

Thiol ligation for the preparation of antibody-drug conjugates is well-known in the art, and may also be referred to as alkylation of a thiol group. It typically takes the form of a nucleophilic reaction, such as a nucleophilic substitution or a Michael reaction. A particular Michael reaction is the maleimide-thiol reaction, which is widely employed in bioconjugation. Thus, in a particular embodiment, Q is reactive in a nucleophilic reaction, preferably in a nucleophilic substitution or a Michael reaction. Herein, it is preferred that Q comprises a maleimide moiety, a haloacetamide moiety, an allenamide moiety, a phosphonamidite moiety, a cyanoethynyl moiety, a vinylsulfone, a vinylpyridine moiety or a methylsulfonylphenyloxadiazole moiety, most preferably a maleimide moiety.

### Thiol-reactive probe Q

Thiol-reactive probe Q is used in the conjugation reaction to connect the linker-drug construct to the antibody of structure AB-((L¹)ₐ-F)x. Q is reactive towards thiol or precursor thereof F in a thiol ligation. Such probes are known in the art and may be selected from the group consisting of a maleimide moiety, a haloacetamide moiety, an allenamide moiety, a phosphonamidite moiety,a cyanoethynyl moiety, a vinylsulfone, a vinylpyridine moiety or a methylsulfonylphenyloxadiazole moiety. Most particularly, Q comprises or is a maleimide moiety.

### Reactive group F

Reactive group F is click probe or a thiol or precursor thereof. Thus, in a first embodiment, F is a click probe. Click probe F is used in the conjugation reaction to connect the linker-drug construct to the modified antibody. F is reactive towards click probe Q in a metal-free click reaction. Such click probes are known in the art and include cyclic alkene, cyclic alkyne, azide, tetrazine, triazine, nitrone, nitrile oxide, nitrile imine, diazo compound, ortho-quinone, dioxothiophene and sydnone.

In particular, F is reactive towards a cyclic alkene, cyclic alkyne, and is typically selected from the group consisting of azide, tetrazine, triazine, nitrone, nitrile oxide, nitrile imine, diazo compound, ortho-quinone, dioxothiophene and sydnone.

In particular, F is reactive towards a diene, such as tetrazines, 1,2-quinones and triazines, and is typically selected from the group consisting of alkene and alkyne group, most particularly an alkyne group or cyclic alkene group.

Particularly, click probe F is selected from azides, tetrazines and cyclic alkenes. Most particularly, click probe F is an azide.

In a second embodiment, F is a thiol or precursor thereof. Thiol or precursor thereof F is used in the conjugation reaction to connect the linker-drug construct to the modified antibody. F is reactive towards thiol-reactive probe Q in a thiol ligation. Thiol precursors in the context of bioconjugation are known in the art, and include disulfides, which may be naturally occurring disulfide bridged present in the antibody or synthetically introduced disulfides, which are reduced as known in the art. Particularly, F is a thiol group.

### The linker-drug construct

In a third aspect, the invention concerns a linker-drug construct according to structure **(5)** 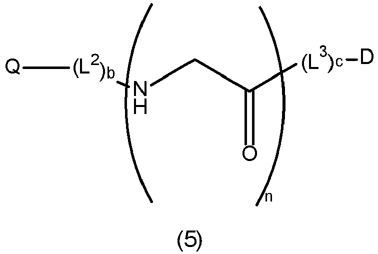 wherein:
Q is a click probe capable of performing a metal-free click reaction or a thiol-reactive probe;
L²,L³,D, n, b and c are as defined herein above

The definitions and preferred embodiments of the linker-drug construct and L2,L3,D, n, b and c provided in the context of the first and second aspect of the invention equally apply to the third aspect of the invention. The linker-drug construct according to this aspect are ideally suitable as intermediate in the preparation of the antibody-drug conjugates according to the present invention. The invention thus also concerns the use of the linker-drug construct according to the invention in a metal-free click conjugation reaction or a thiol ligation conjugation reaction with a modified antibody of structure AB-((L¹)c-F)x.

### Application

The invention further concerns the use of the conjugate according to the invention in medicine.

In a further aspect, the present invention provides a method of treating a subject in need thereof, comprising administering the conjugate according to the invention to the subject.

In a further aspect the present invention provides the conjugate according to the invention for use in treatment, in particular for use in the treatment of a subject in need thereof.

In a further aspect, the present invention provides the use of the conjugate according to the invention in the manufacture of a medicament.

The administration typically occurs with a therapeutically effective amount of the conjugate according to the invention.

The invention further provides a method for the treatment of a specific disease in a subject in need thereof, comprising the administration of the conjugate according to the invention as defined above. In particular, the specific disease may be selected from cancer and an autoimmune disease, particularly the disease is cancer. The subject in need thereof is typically a cancer patient. The use of antibody-drug conjugates is well-known in such treatments, particularly in the field of cancer treatment, and the conjugates according to the invention are particularly suited in this respect. In the method according to this aspect, the conjugate is typically administered in a therapeutically effective amount.

In a further aspect, the present invention provides a conjugate according to the invention for use in the treatment of a specific disease in a subject in need thereof, particularly for the treatment of cancer.

In a further aspect, the present invention provides the use of a conjugate according to the invention for the preparation of a medicament or pharmaceutical composition for use in the treatment of a specific disease in a subject in need thereof, preferably for use in the treatment of cancer.

Administration in the context of the present invention refers to systemic administration. Hence, in one embodiment, the methods defined herein are for systemic administration of the conjugate. In view of the specificity of the conjugates, they can be systemically administered, and yet exert their activity in or near the tissue of interest (e.g. a tumour). Systemic administration has a great advantage over local administration, as the drug may also reach tumour metastasis not detectable with imaging techniques and it may be applicable to haematological tumours.

The invention further provides a pharmaceutical composition comprising the antibody- payload conjugate according to the invention and a pharmaceutically acceptable carrier.

Some aspects and embodiments of the disclosure are described below in more detail with reference to the following examples, which are illustrative only and non-limiting.

The examples refer to the following figure:

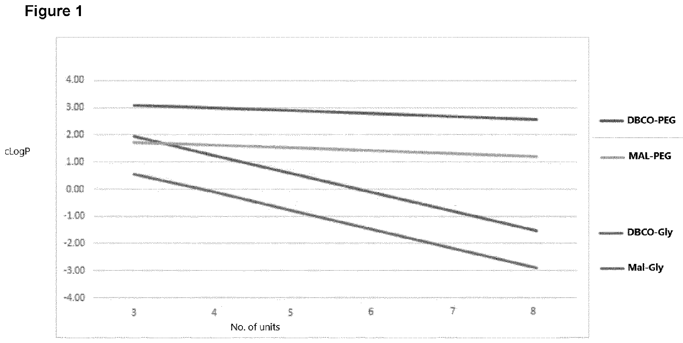

- Figure 1: - shows a plot of cLogP against number of units of glycine or PEG.

### EXAMPLES

### Materials and Methods

### Preparation of Drug Linkers

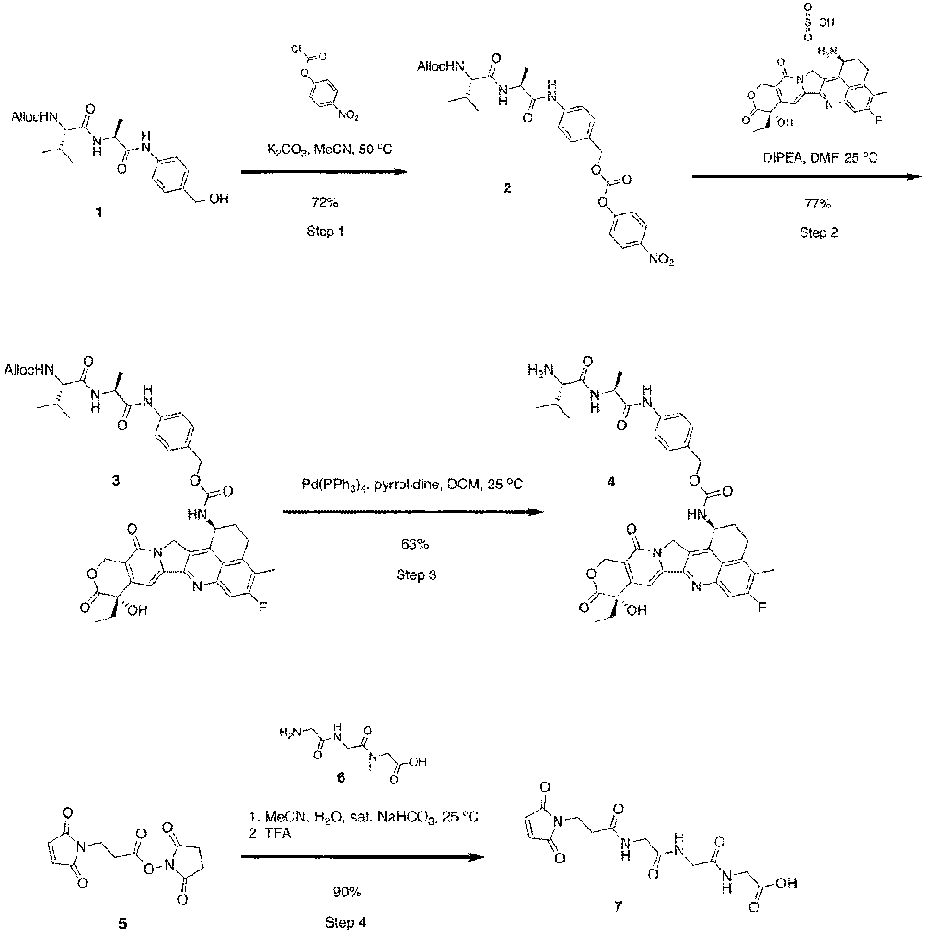

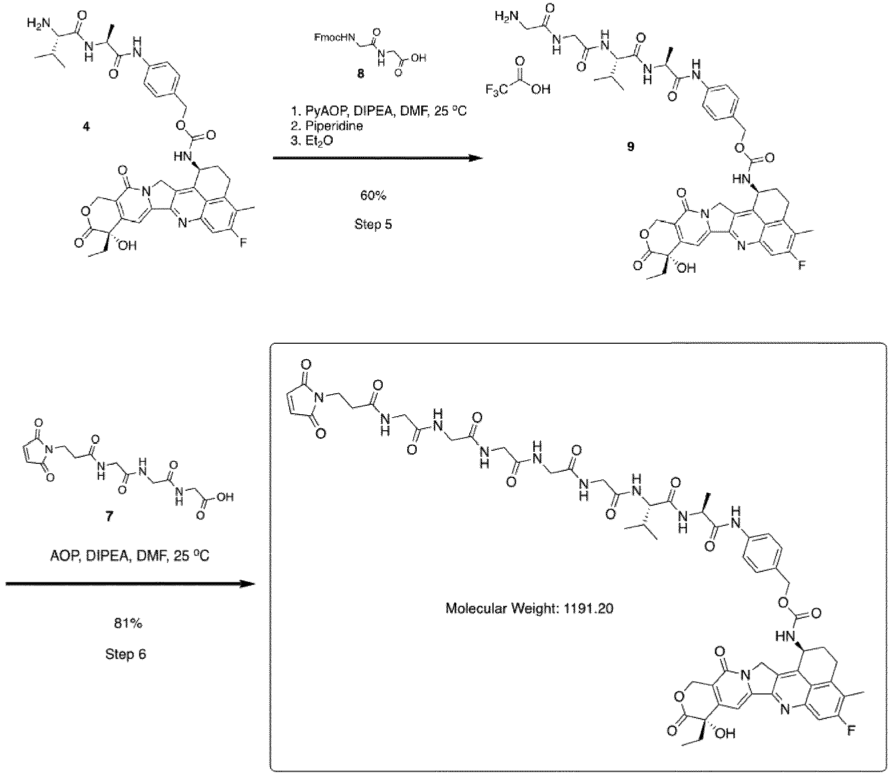

### Step 1:

Alloc-Val-Ala-PABA (1) (29 g, 1 eq) was dissolved in MeCN (10 V). K₂CO₃ (2 eq) was added. The reaction mixture was heated to 50°C. A solution of 4-nitrophenyl carbonochloridate in MeCN (2 eq in 5 V) was added. The reaction mixture was stirred at 50 °C for 6 h. On completion, the reaction mixture was filtered. The filtrate was concentrated in vacuo to give the crude residue. The latter was purified by flash column chromatography (silica) eluting with EtOAc/DCM (0 -> 20%) to afford compound 2 (30 g, 72%).

### Step 2:

Exatecan mesylate (24 g, 1 eq) was dissolved in DMF (10 V). DIPEA (3 eq) was added. Compound **2** (1.25 eq) was added and the reaction mixture was stirred at 25°C for 16 h.

On completion, water (30 V) was added dropwise to the reaction mixture. The latter was stirred at 25 °C for 30 min. The reaction mixture was filtered and the cake was washed with water (5 V), collected and thoroughly dried to afford compound **3** (29 g, 77%).

### Step 3:

Compound **3** (30 g, 1 eq) was dissolved in DCM (10 V). Pd(PPh₃)₄ (0.02 eq) and pyrrolidine (1.5 eq) were added. The reaction mixture was stirred at 25°C for 2 h. On completion, the reaction mixture was concentrated in vacuo to give the crude residue. The latter was purified by flash column chromatography (silica) eluting with MeOH/DCM (0 -> 8%) to afford compound **4** (17 g, 63%).

### Step 4:

To a solution of compound **5** (5.32 g, 20 mmol) and compound 6 (3.8 g, 20 mmol) in acetonitrile/water (1/2, v/v, 100 mL) was added sat. NaHCO₃ solution (40 mL).

The reaction was stirred at room temperature for 2 h. Then TFA (2.8 mL) was added and the stirring continued for an additional 10 min. The mixture was purified directly by preparative RP-HPLC to afford compound 7 as a white solid (6.1 g, 90%).

### Step 5:

To a solution of compound **4** (3.08 g, 4.08 mmol) and compound 8 (1.44 g, 4.08 mmol) in anhydrous DMF (50 mL) was added PyAOP (2.13 g, 4.08 mmol) and DIPEA
(2.1 mL, 12.08 mmol). The reaction was stirred at room temperature for 10 min. Then piperidine (2.0 mL) was added and the stirring continued for an additional 10 min.

The mixture was added to diethyl ether (500 mL) while stirring. The suspension was centrifugated and the precipitate was collected by decantation. Then the precipitate was dissolved in 40 mL of DMF and purified by preparative RP-HPLC to give compound 9 as a yellow solid, TFA salt (2.4 g, 60%).

### Step 6:

To a solution of compound 9 (TFA salt, 814 mg, 0.83 mmol) and compound 7 (282 mg, 0.83 mmol) in anhydrous DMF (20 mL) was added AOP (367 mg, 0.83 mmol) and DIPEA (0.57 mL, 3.28 mmol). The reaction was stirred at room temperature for 10 min. Then the mixture was purified by preparative RP-HPLC to afford compound DL-A as a yellow solid (510 mg, 81%).

### Preparative RP-HPLC method:

Column: Phenomenex Gemini NX 5m, C18, 110Å, 150 × 50 mm
Instrument: Shimadzu LC with CTC IFC
Mobile phase: A) water (0.1% TFA), B) acetonitrile
Gradient: 10-65% B over 20 min, flow 50 mL/min

### Analytical HPLC:

Column: Phenomenex Gemini NX 5m, C18, 110 Å, 150 × 4.6 mm
Instrument: Shimadzu LC-2030
Mobile phase: A) water (0.05% TFA), B) acetonitrile (0.05% TFA)
Gradient: 20-70% B over 15 min, flow 1.0 mL/min

All the desired HPLC fractions were pooled and lyophilised on a Virtis Freezemobile 35EL.

### Synthesis of ADCs

The test ADCs have Exatecan as the warhead with a maleimide-Gly5 (or Gly3)-Val-Ala-PABA linker (DL-A) conjugated to a test monoclonal antibody via hinge region cysteine resides essentially as described in Zammarchi et al., 2016. Mol Cancer Ther 15: 2709. In brief, antibody was buffer exchanged into a histidine buffer at pH 6 using tangential flow filtration, pH was adjusted to 7.5 using a TRIS/EDTA pH 8.5 buffer, and the solution was reduced with Tris (2-carboxyethyl) phosphine reductant (TCEP). Dimethylacetamide and drug-linker (threefold excess relative to antibody) were added to the solution. The conjugation reaction was incubated, then quenched with threefold molar excess of N-acetyl cysteine and incubated again. The pH was then decreased to 6.0 using histidine hydrochloride solution and the generated ADC was purified by tangential flow filtration, filtered, and stored at -70°C. Final yield was estimated by ultraviolet-visible spectrophotometry based on starting antibody. Synthesis of the drug linker DL-A is described above.

### Aggregation Studies

2 mg of test monoclonal antibody at 8.87 mg/ml was pH adjusted with 5% 0.5 M Tris, 0.025 M EDTA, pH 8.5 and reduced with 2.3 mol. eq. TCEP and then conjugated with 6mol. eq. drug linker. After buffer exchange into 30mM Histidine, 175 mM Sucrose pH 6.0/PBS, the drug to antibody ratio (DAR) was by HIC/LC-MS and %Monomer by size exclusion chromatography (SEC). Percentage monomer values of 95% or greater are assessed as low aggregation.

### Example 1 - Aggregation Studies with Exatecan linkers

Previous work that we have conducted using PEG-based linkers and an Exatecan warhead showed unfavourable aggregation properties. Therefore we sought to identify an alternative linker design. A new linker was designed using multiple glycine residues instead of PEG and this led to an improvement in levels of aggregation (data not shown). This glycine linker concept was then tested in new constructs based on a maleimide conjugation group and a Val-Ala-PABC cleavable portion linked to Exatecan.

A maleimide-Gly3-Val-Ala-PABC-Exatecan construct was tested against a maleimide-Gly5-Val-Ala-PABC-Exatecan construct (DL-A, the synthesis of which is described above - the Gly3 variant differs only with respect to the number of Gly residues). LogP values (a measure of hydrophobicity) were estimated using CDD Vault software (Collaborative Drug Discovery Inc.) based on the method described in Gedeck et al., 2017, J. Chem. Inf. Model. 57, 8, 1847-1858. Model training information using publicly available datasets is available from CDD. The results are shown in Table 1.

A correlation study was also carried out using Reverse-Phase HPLC (RP-HPLC). The two constructs were injected in RP-HPLC and retention times (Rt) correlated with hydrophobicity - i.e., high Rt was associated to hydrophobic constructs whereas low Rt to hydrophilic ones. The results are shown in Table 1.

Aggregation levels were assessed during the conjugation to the test antibody.

During conjugation, we are ideally targeting a monomer content or percentage monomer of 95% or above. Low percentage monomer values usually translate to a higher degree of aggregation. One could therefore agree that percentage monomer values of 95% or higher mean very low levels of aggregation or none, whereas values below this threshold often translate to higher levels of aggregation.

### Results

**Table 1**

| Construct ID | cLogP | Rt (min) | Aggregation (% monomer) |
|---|---|---|---|
| Mal-Gly5-VA-PABC-Exatecan | -0.81 | 9.76 | None (98%) |
| Mal-Gly3-VA-PABC-Exatecan | 0.59 | 9.98 | Moderate (87%) |

### Discussion

These results clearly show the hydrophilicity-enhancing properties in a drug linker construct of a novel pentaglycine spacer/linker. The incorporation of this Gly5 motif allowed constructs to exhibit increased hydrophilicity and lower aggregation levels for optimal conjugation processes. Gly5-based constructs showed no aggregation versus moderate aggregation for a Gly3-based version.

### Example 2 - Comparison of cLogP values for different polyglycine and PEG linkers.

cLogP values were calculated as described above for polyglycine and polyethyleneglycol (PEG) linkers with lengths of from 5 to 8, with two different conjugation groups - maleimide (Mal) and dibenzocyclooctyne (DBCO). PEG is commonly used in linkers to enhance hydrophilicity. The values are shown below in Table 2.

**Table 2**

| | Mal-Glyn | DBCO-Glyn | Mal-PEGₙ | DBCO-PEGₙ |
|---|---|---|---|---|
| 3.00 | 0.56 | 1.95 | 1.73 | 3.09 |
| 4.00 | -0.11 | 1.25 | 1.62 | 2.99 |
| 5.00 | -0.81 | 0.56 | 1.52 | 2.88 |
| 8.00 | -2.9 | -1.53 | 1.2 | 2.56 |

These results are plotted in Figure 1. It can readily be seen that for the same number of units, glycine results in a lower cLogP. In addition, glycine has a stronger effect on lowering cLogP per unit added.

### Example 3 - Conjugation Studies with Exatecan linkers - PEG versus Glycine

Both Mal-Gly5-VA-PABC-Exatecan and a variant with PEG5 instead of Gly5 were conjugated to the same test antibody essentially as described in Example 1 but with different cysteine mutations that give rise to different target maximum drug to antibody ratios (DARs) - (4, 6 or 8). The resulting conjugates were analysed by HIC-UHPLC (TSKgel Butyl-NPR column) and PLRP-HPLC (MAbPAC RP) to determine the extent of conjugation.

### Results

**Table 3**

| | **Payload** | **TCEP eq.** | **Payload eq.** | **DAR (HIC)** | **DAR (PLRP)** |
|---|---|---|---|---|---|
| **Target DAR 8** | | | | | |
| | Mal-Gly5-VA-PABC-Exatecan | 7.5 | 10.0 | 7.3 | 7.9 |
| | Mal-PEG5-VA-PABC-Exatecan | 7.5 | 10.0 | 5.9 | 5.9 |
| **Target DAR 6** | | | | | |
| | Mal-Gly5-VA-PABC-Exatecan | 6.0 | 8.0 | 5.4 | 6.0 |
| | Mal-PEG5-VA-PABC-Exatecan | 6.0 | 8.0 | 3.1 | 3.3 |
| **Target DAR 4** | | | | | |
| | Mal-Gly5-VA-PABC-Exatecan | 3.0 | 6.0 | 4.0 | 3.8 |
| | Mal-PEG5-VA-PABC-Exatecan | 3.0 | 6.0 | 0.8 | 0.7 |

### Conclusions:

The Gly₅ version led to much better conjugation profiles than the PEG₅ version.

A number of publications are cited above to more fully describe and disclose the disclosures and the state of the art to which inventions herein may pertain. The entirety of each of the references mentioned in this disclosure are hereby is incorporated by reference.

## Claims

1. An antibody-drug conjugate, having structure (1) 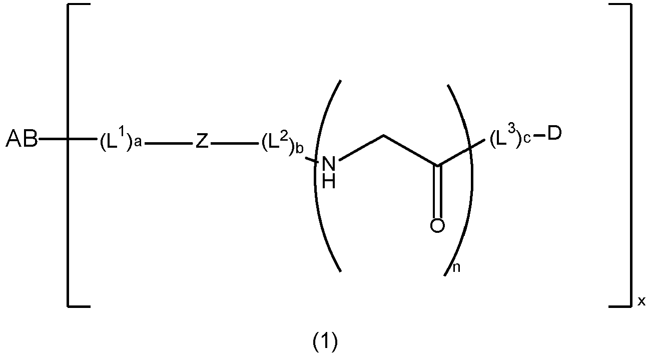 wherein:
AB a cell binding agent such as an antibody;
n is an integer in the range of 5 - 8;
L¹ is a linker;
L² is a linker;
L³ is a linker;
a, b and c are independently 0 or 1;
Z is a connecting group obtained by a metal-free click reaction or by thiol ligation;
or Z is absent wherein a and b are 0 and the N terminus of the polyglycine is bonded directly to AB;
D is a drug payload;
x is an integer in the range of 1 - 8.

2. An antibody-drug conjugate accordingly to claim 1 wherein L³ comprises a cathepsin cleavable peptide.

3. An antibody-drug conjugate accordingly to claim 1 or 2, wherein L³ is 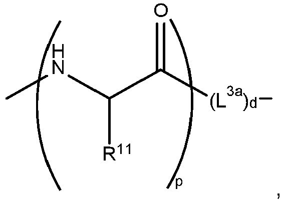 having structure (1a) 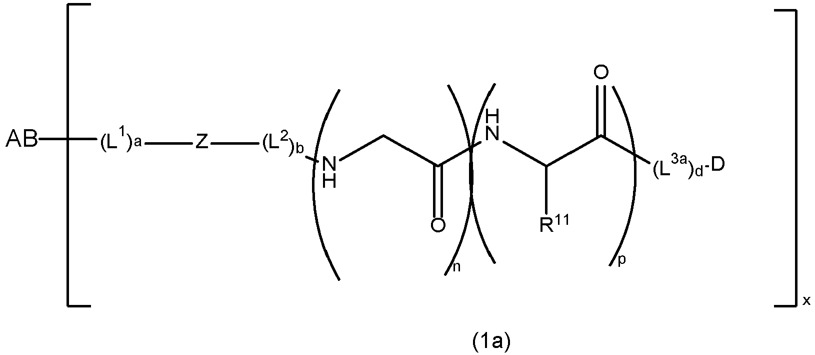 wherein:
AB is a cell binding agent such as an antibody;
n is an integer in the range of 5 - 8;
L¹ is a linker;
L² is a linker;
L^{3a} is a linker;
a, b and d are independently 0 or 1;
Z is a connecting group obtained by a metal-free click reaction or by thiol ligation;
or Z is absent wherein a and b are 0 and the N terminus of the polyglycine is conjugated directly to AB;
D is a drug payload;
p is an integer in the range 1 to 5;
each R¹¹ is independently an amino acid side chain;
x is an integer in the range of 1 - 8.

4. An antibody-drug conjugate according to any preceding claim wherein L³ comprises a self-immolative group.

5. An antibody-drug conjugate according to claim 3 or 4 having structure (1b) 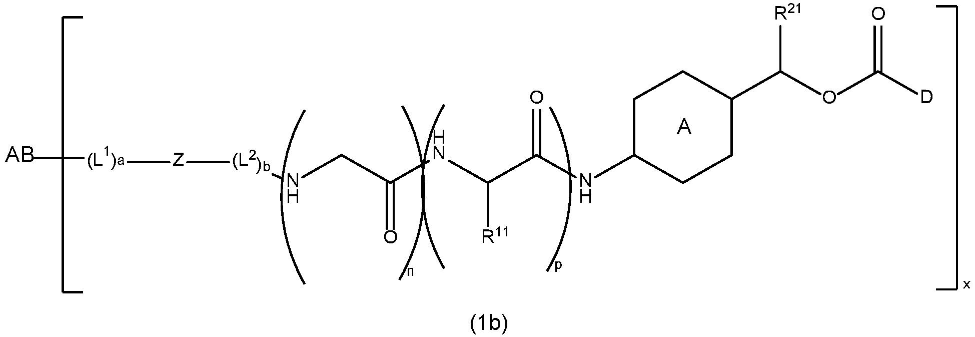 wherein:
AB is a cell binding agent such as an antibody;
n is an integer in the range of 5 - 8;
L¹ is a linker;
L² is a linker;
L^{3a} is a linker;
a and b are independently 0 or 1;
Z is a connecting group obtained by a metal-free click reaction or by thiol ligation;
or Z is absent wherein a and b are 0 and the N terminus of the polyglycine is bonded directly to AB;
D is a drug payload;
p is an integer in the range 0 to 5;
each R¹¹ is independently an amino acid side chain;
A is an optionally substituted 5- or 6-membered aromatic or heteroaromatic ring;
R²¹ is selected from H, R²², C(O)OH and C(O)R²², wherein R²² is C₁ - C₂₄ (hetero)alkyl groups,
C₃ - C₁₀ (hetero)cycloalkyl groups, C₂ - C₁₀ (hetero)aryl groups, C₃ - C₁₀ alkyl(hetero)aryl groups and C₃ - C₁₀ (hetero)arylalkyl groups, which are optionally substituted and optionally interrupted by one or more heteroatoms selected from O, S and NR²³ wherein R²³ is independently selected from the group consisting of hydrogen and C₁ - C₄ alkyl groups.
x is an integer in the range of 1 - 8.

6. The antibody-drug conjugate according to any preceding claim, wherein each occurrence of the peptide (NH-CR¹¹-CO)p is selected from Val-Cit, Glu-Val-Cit, Val-Ala, Val-Lys, Val-Arg, Aclys-Val-Cit, Aclys-Val-Ala, Glu-Val-Ala, Asp-Val-Ala, Phe-Gly, Phe-Cit, Phe-Ala, Phe-Lys, Phe-Arg, Ala-Lys, Leu-Cit, Ile-Cit, Trp-Cit, Ala-Ala-Asn, Ala-Asn and Lys, preferably from Val-Cit, Glu-Val-Cit, Val-Ala, Glu-Val-Ala, Val-Lys, Phe-Gly. Phe-Cit, Phe-Ala, Phe-Lys, Ala-Ala-Asn, most preferably Val-Cit or Val-Ala.

7. The antibody-drug conjugate according to any preceding claim, wherein connecting group Z is obtainable by a metal-free click reaction.and comprises a triazole moiety; or group Z is obtainable by thiol ligation comprises a succinimidyl ring or its ring- opened succinic acid amide derivative,

8. The antibody-drug conjugate according to any preceding claim, which has structure (1c) 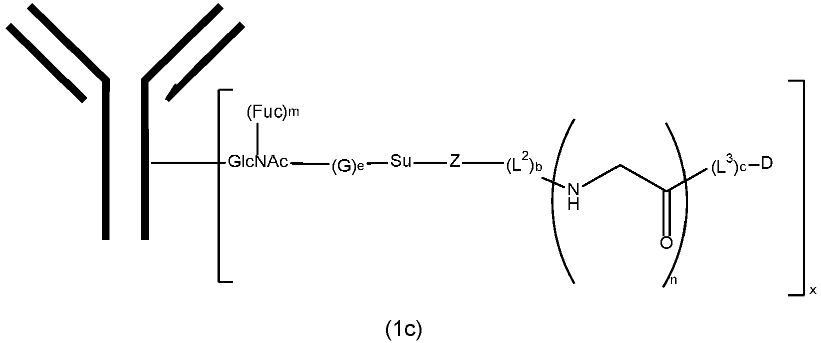
Z, L², L³, D, b, c, n and x are as defined herein;
e is an integer in the range of 0 - 20;
Su is a monosaccharide;
G is a monosaccharide moiety;
GlcNAc is an N-acetylglucosamine moiety;
Fuc is a fucose moiety;
m is 0 or 1.

9. A process for the synthesis of the antibody-drug conjugate according to any one of claims 1 - 7, comprising reacting
(i) a modified antibody of structure AB-((L¹)ₐ-F)x, wherein:
AB a cell binding agent such as an antibody;
L¹ is a linker;
a is 0 or 1;
F is a click probe capable of reacting with Q in a metal-free click reaction or a thiol or
precursor thereof
x is an integer in the range of 1 - 8;
with
a linker-drug construct according to structure (5): 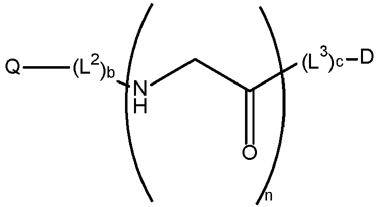
wherein:
Q is a click probe capable of performing a metal-free click reaction or a thiol-reactive probe;
L²,L³,D, n, b and c are as defined in any preceding claim;
to form an antibody-drug conjugate wherein the drug is covalently attached to the antibody via connecting group Z that is formed by a metal-free click reaction between Q and F or by thiol ligation between Q and F.

10. The process according to claim 8, wherein the click probe Q comprises a cyclic alkyne moiety, cyclic alkene moiety.or a tetrazine moiety.

11. The process according to claim 8, wherein the thiol-reactive probe Q is comprises or is a maleimide moiety.

12. The process according to any one of claims 8 - 11, wherein click probe F is selected from the group consisting of azides, tetrazines, triazines, nitronse, nitrile oxides, nitrile imines, diazo compounds, ortho-quinones, dioxothiophenes, sydnones, alkynes and cyclic alkenes, preferably click probe F is an azide moiety; and/or wherein the click reaction is a 1,3- dipolar cycloaddition or a (4+2) cycloaddition; or F is a thiol; and/or wherein the thiol ligation is a Michael addition or a nucleophilic substitution.

13. The process according to any one of claims 8 - 11, wherein AB-((L¹)a-F)x is according to structure (6): 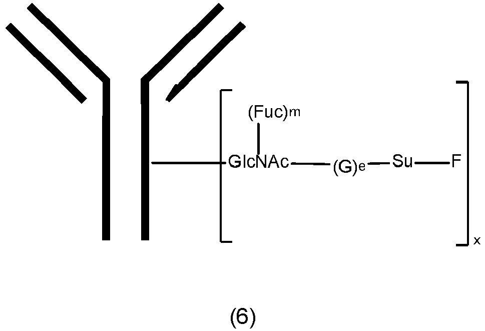 wherein:
e is an integer in the range of 0- 10;
Su is a monosaccharide;
G is a monosaccharide moiety;
GlcNAc is an N-acetylglucosamine moiety;
Fuc is a fucose moiety;
m is 0 or 1.

14. A linker-drug construct according to structure (5): 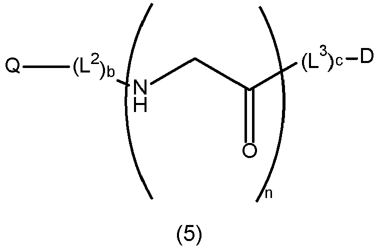 wherein:
Q is a click probe capable of performing a metal-free click reaction or a thiol-reactive probe;
AB is an antibody;
n is an integer in the range of 5 - 8;
L² is a linker;
L³ is a linker;
b and c are independently 0 or 1;
L²,L³,D, n, b and c are as defined in any preceding claim.

15. The linker-drug construct according to claim 13, wherein the click probe Q comprises a cyclic alkyne moiety or a cyclic alkene moiety

16. The linker-drug construct according to claim 12, wherein the thiol-reactive probe Q comprises or is a maleimide moiety..

17. Pharmaceutical composition comprising the antibody-drug conjugate according to any one of claims 1 - 7 and a pharmaceutically acceptable carrier.

18. The antibody-drug conjugate according to any one of claims 1 - 7, for use in the treatment of a subject in need thereof.
